# FASCICULE DE BREVET EUROPEEN

(11) **EP 0 414 855 B1**
(45) Date de publication et mention de la délivrance du brevet: **10.11.1993**
(21) Numéro de dépôt: 90903179.1
(22) Date de dépôt: 02.02.1990
(51) Int. Cl.: A61M 3/02

(54) **APPAREIL D'IRRIGATION ET D'ASPIRATION UTILISABLE EN CHIRURGIE ENDOSCOPIQUE**
IRRIGATIONS- UND ASPIRATIONSVORRICHTUNG ZUR VERWENDUNG BEI DER ENDOSKOPISCHEN CHIRURGIE
IRRIGATION AND SUCTION APPARATUS USABLE IN ENDOSCOPIC SURGERY

(30) Priorité: 02.02.1989 FR 8901648
(43) Date de publication de la demande: 06.03.1991
(73) Titulaire: SINERGY S.A., F-03300 Cusset (FR)
(72) Inventeur: OGNIER, Jean-François, F-15240 Saignes (FR); MANHES, Hubert, F-03200 Vichy (FR)
(74) Mandataire: Chanet, Jacques
(86) Numéro de dépôt international: FR9000086
(87) Numéro de publication internationale: WO9008562

(56) Documents cités:
- WO-A-87/00759
- BE-A- 403 715
- FR-A- 2 378 494
- GB-A- 2 205 244
- US-A- 2 112 145
- US-A- 3 177 871
- US-A- 3 900 022
- US-A- 4 486 389
- US-A- 4 702 733

## Description

La présente invention est du domaine des techniques chirurgicales et elle a plus particulièrement pour objet un appareil d'irrigation et d'aspiration utilisable en chirurgie endoscopique.

On rappelle que la chirurgie, et/ou l'exploration, endoscopique met généralement en oeuvre des moyens d'irrigation assurant le nettoyage du champs opératoire, des moyens d'aspiration assurant l'évacuation des liquides et des tissus corporels, et des moyens de dilatation, ou de mise en tension, des organes ou de la cavité siège de l'intervention. L'irrigation et la dilatation peuvent être réalisées simultanément par injection sous légère pression d'un soluté isotonique ou très légèrement hypotonique, tel qu'avantageusement le glycocolle en raison de ses propriétés diélectriques. On rappelle, à propos de cette technique chirurgicale, qu'il est impératif de maintenir la surpression entre un seuil minimal (suffisant pour dilater la cavité et contrebalancer la pression capilaire) et un seuil maximal (correspondant à la pression d'absoption par le système veineux, du liquide d'irrigation). Ces seuils, variables suivant les individus, se situent respectivement à environ 2,0 et 6,0 kPa (15 et 45 mmHg).

Des techniques d'irrigation ont été largement décrites, notament dans les publications de brevet, et on pourra avantageusement se reporter aux brevets FR 2244440 (IGLESIAS), et FR 256954, FR 2569555 (BURNER). Le premier de ces brevets décrit des moyens simples d'alimentation en fluide d'irrigation constitués essentiellement par un réservoir en charge et par des robinets pour le contrôle du débit. Les brevets BURNER décrivent des moyens plus élaborés mettant notament en oeuvre des moyens de régulation automatique de la pression grâce à une pompe placée sous la dépendance d'un manostat. Le second de ces derniers brevets décrit plus particulièrement une technique d'aspiration mettant en oeuvre des moyens de régulation de la pression. On notera cependant que même avec des appareils à pression régulée, on n'est pas totalement prémuni contre les risques de passage du liquide dans le réseau sanguin, en raison des variations importantes de la pression artérielle qui peuvent avoir lieu au cours de l'intervention.

On connaît aussi par les publications WO 8700759 (MINNESOTA), FR 2378494 (WOLF) et US 3900022 (WIDRAN) un dispositif d'irrigation pour injecter du liquide d'irrigation dans une cavité opératoire ; cependant ces dispositifs ne comportent pas de moyens de chauffage du liquide ni de contrôle de sa température. On pourra aussi, pour connaître des techniques annexes, se référer aux publications suivantes : US 4486384 (DARNELL), GB 2205244 (BLOGOVESCHEN-SKY), US 3177871 (MYERS) et US 4702733 (WRIGHT).

On rappelle enfin que l'on connait bien l'influence hémostatique de la température, en notant cependant que cette influence résulte de deux causes à effets contraires ; d'une part une cause à effet positif consistant en l'augmentation de la vitesse de polymérisation des fibrinogènes et de la vitesse de formation du "clou plaquettaire" d'autre part une cause à effet négatif consistant en la vasodilatation.

A la connaissance des demandeurs on n'a jamais cherché, en chirurgie laparotomique mettant en oeuvre un sérum d'irrigation, à exploiter l'influence hémostatique de la seule température du sérum chaud, en raison de la prédominance de l'effet négatif ; en particulier on a pu utiliser du sérum à 40-45°C, mais après avoir pincé ou suturé les vaisseaux coupés. Cependant les Demandeurs ont pensé qu'il pouvait ne pas en être de même en chirurgie endoscopique, en raison de l'effet positif de la mise en surpression de la cavité opératoire. Leurs travaux les ont ainsi amenés à constater qu'un sérum, ou autre liquide d'irrigation, injecté à une température comprise entre environ 42 et 45°C ralentissait de façon considérable le flux hémoragique ; globalement l'apport de sérum isotonique chaud (42-45°C) au lieu du sérum à température ambiante de la pratique antérieure, a pour conséquences : d'accélèrer l'hémostase des vaisseaux en nappe, de permettre la réduction du flux d'irrigation nécessaire au nettoyage du champ opératoire et à l'entretien d'une parfaite vision. Il restait encore le problème délicat des risques de transfert du liquide dans le système sanguin, avec les risques pre et post-opératoires qu'il entraine, problème pour lequel la technique de l'invention apporte une solution. Partant de ces observations, l'invention propose des moyens assurant de façon optimale l'irrigation du champ opératoire en chirurgie endoscopique, avec une limitation maximale du risque de passage du liquide dans le réseau sanguin.

La présente invention propose un appareil d'irrigation et d'aspiration pour la chirurgie endoscopique qui est defini, dans sa disposition principale, par la revendication 1.

Suivant une disposition secondaire le dispositif de l'invention comprend aussi des moyens de mesure différentielle entre les volumes injectés et les volumes récupérés ; des moyens peuvent avantageusement être prévus pour traiter ces mesures et fournir une mesure différentielle instantanée, des mesures cumulées, voire pour intervenir sur les moyens générateurs de la surpression dans la cavité. Ainsi le sérum passe successivement du réservoir dans une pompe péristaltique, puis dans la cavité par l'intermédiaire de l'instrument, puis dans une cellule piézométrique mesurant la surpression dans la cavité, puis dans une vanne de contrôle de la pression intracavitaire, pour être finalement receptionné dans une enceinte en dépression.

Avantageusement l'entrainement de la pompe péristaltique est placé sous la dépendance à la fois de moyens manuels de réglage du débit, et de moyens automatiques d'arrêt en cas de surpression dans la cavité, tandis que la vanne de contrôle de la pression intracavitaire est placée sous la dependance des moyens piézométriques de mesure de la surpression dans la cavité ; la pompe et/ou la vanne pourront éventuellement, comme indiqué plus haut, être aussi placées sous la dépendance des moyens de mesure ou de calcul des débits de liquide

Selon une forme particulière de réalisation; la vanne de réglage est du type à écrasement de tuyau par un doigt presseur, le dit doigt presseur étant mû par un vérin pneumatique actionné en ouverture par la dépression régnant dans ladite enceinte, et en fermeture par un ressort agissant à l'encontre de la dépression.

Selon une autre disposition particulière de réalisation; les moyens de chauffage et/ou de maintien en température sont constitués par un bain-marie dans lequel baigne au moins une poche de contention du liquide d'irrigation ; avantageusement cependant le bain-marie est prévu suffisament grand pour pouvoir contenir plusieurs poches, par exemple deux poches de liquide dit "physiologique" et deux poches de liquide dit de Ringer.

Suivant une autre disposition particulière de réalisation, un moyen de chauffage dudit bain-marie est constitué par une résistance électrique placée sous la dépendance d'un thermostat de régulation à seuil haut et à seuil bas distants en température d'environ 2°C, ces seuils étant réglables. A titre d'exemple on pourra choisir un seuil bas de déclenchement à 42°C et un seuil haut d'arrêt à 44°C. Il pourra être prévu également un thermostat de sécurité avec un seuil haut d'arrêt à 50°C.

Enfin, et de manière avantageuse, un tube souple continu relie ladite poche baignant dans le bain-marie, à un ajutage d'entrée dudit instrument, le dit tube souple continu constituant le tuyau de la pompe péristaltique ; le réservoir étant avantageusement une poche souple.

Suivant une disposition avantageuse le tube souple destiné à relier la poche à l'instument, constitue avec la poche un conditionnement stérile du liquide d'irrigation, et, le tube souple reliant l'enceinte de réception à la dite vanne de contrôle, d'une part, et à une pompe à vide d'autre part, constitué avec l'enceinte un conditionnement stérile du liquide d'irrigation récupéré.

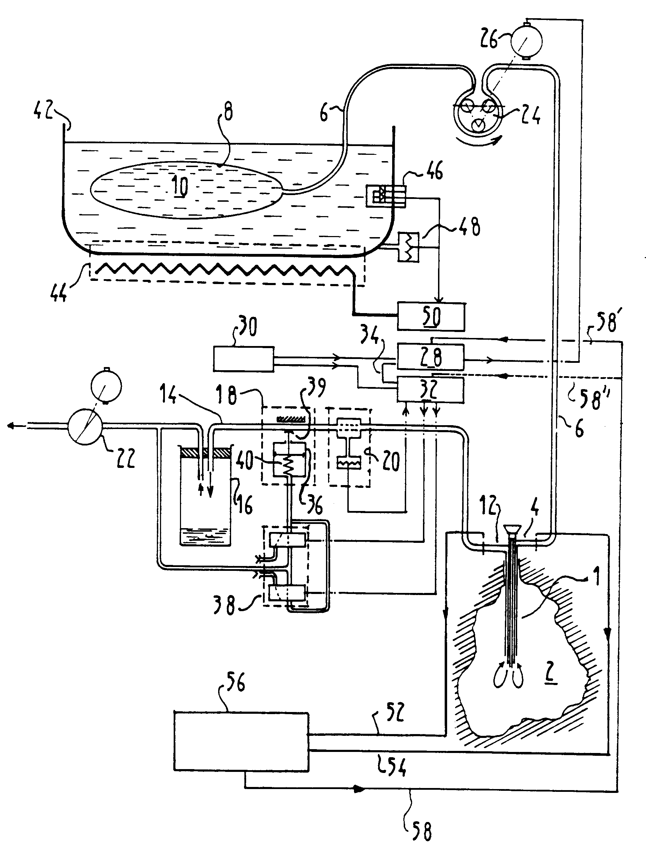

La présente invention sera mieux comprise, et des détails en relevant apparaîtront, à la description qui va être faite d'une forme particulière et préférée de réalisation de l'invention, en relation avec la figure de la planche unique annexée qui est une représentation schématique de l'appareil conforme à l'invention.

Sur la figure, un instrument de chirurgie endoscopique 1, par exemple un instrument du genre décrit dans une Demande de brevet FR 8800170, est introduit dans une cavité corporelle 2 ; un premier ajutage 4 de l'instrument est relié par un premier tube souple continu 6 à une poche souple 8, constituant le réservoir cité plus haut, et contenant un liquide 10 d'irrigation, tandis qu'un second ajutage 12 de sortie est relié par un second tube souple 14 à une enceinte de reception 16 en dépression ; une vanne 18 à écrasement de tuyau est interposée sur le second tube souple entre la sortie 12 de l'instrument et l'enceinte 16, tandis qu'une cellule piézométrique 20 est traversée par le liquide en aval de l'instrument et en amont de la vanne. Une pompe à vide 22 établit une forte dépression dans l'enceinte 16.

Une pompe péristaltique 24 entraînée par un moteur 26 agit sur le tube souple pour transferer le liquide 10 de la poche 8 vers la cavité 2 ; le moteur d'entrainement 26 de la pompe 24 est contrôlé en rotation par des moyens 28 réglables manuellement à partir d'un clavier 30 ; le débit de la pompe 26 est réglable de 0 à 700 ml/min.

Le clavier 30 permet aussi le réglage par les moyens de contrôle 32 de la surpression dans la cavité 2, les moyens de contrôle 32 étant en relation avec la cellule piézométrique 20 ; on remarque aussi que les moyens de contrôle 32 ont aussi une relation 34 avec les moyens 28 de contrôle de vitesse de rotation de la pompe, pour arrêter celle ci en cas de dépassement d'un seuil de surpression (environ 6,6 kPa (50 mmHg)) dans la cavité 2.

La vanne 18 est constituée par la paroi souple du tube 14 et par un doigt 39 monté en bout de tige d'un vérin 36 ; cette disposition assure des conditions de stérilité convenables en aval de la cavité ; une distribution 38 à commande electromagnétique met le vérin en communication soit avec le l'atmosphère, soit avec l'entrée de la pompe à vide 22 ; un ressort 40 sollicite le doigt en position de fermeture. Il doit être compris que tout autre mécanisme opérant la manoeurvre du doigt presseur, par exemple électromagnétique, mécanique, à commande numérique ou analogique, pourrait remplacer le mécanisme pneumatique précité.

La poche 8 barbote librement dans le bain-marie 42 ; celui-ci est chauffé par une résistance électrique 44 sous la dépendance de moyens de régulation en température 50 comprenant un couple 46 de thermistances (ou autre dispositf thermométrique à deux seuils) ; des moyens 48, piézométriques par exemple, peuvent interdire le chauffage en cas d'insuffisance d'eau dans le bain-marie.

Suivant une disposition alternative intéressante, la poche 8 et le tube souple 6 constituent un conditionnement stérile du liquide qu'ils contiennent, lors de la mise à disposition du praticien, lequel a à sa charge de le brancher de façon stérile sur l'instrument, et d'engager la partie médiane du tube dans la pompe 24.

On a représenté par les lignes 52 et 54 des lignes de transmission de signaux en provenance de capteurs de débit disposés à l'entrée et à la sortie de l'instrument 1 au voisinage des ajutages 4 et 12, et en direction d'un ensemble de controle 56. Il s'agira de préférence de capteurs de débit sans contact avec le fluide en écoulement, tels que par exemple des capteurs électromagnétiques ou ultrasonores ; l'ensemble 56 fournit la lecture des débits instantanés, intègre leur différence et cumule ces mesures et leur différence, pour fournir une possibilité de contrôle d'un passage éventuel, instantané ou cumulé, de liquide dans le réseau sanguin. A travers une ligne 58(58',58'') l'ensemble 56 peut agir, en fonction des mesures volumétriques, sur les moyens de commande 28 de la pompe 24, et/ou sur les moyens de commande 32 de la vanne 18 ; l'ensemble de controle 56 peut incorporer des moyens de signalisation sensible, moyens sonore par exemple, pour indiquer instantanément au praticien le franchissement de certains seuils de débit.

Une autre façon simple de mesurer un passage éventuel de liquide d'irrigation dans le réseau sanguin consiste, toujours selon l'invention, en la pesée précise et permanente, par exemple par jauges de contraintes, de l'ensemble de l'appareil et du liquide, en amont et en aval de l'instrument : en l'absence de passage du liquide vers le réseau sanguin, le poids de l'ensemble doit être constant ; des moyens de signalisation analogues à ceux utilisés avec la méthode volumétrique sus-décrite, peuvent être utilisés avec cette méthode gravimétrique.

Bien que l'on ait décrit et représenté une forme particulière de l'appareil d'irrigation de l'invention, il doit être compris que la portée de cette dernière n'est pas limitée à cette forme, mais qu'elle s'étend à tout appareil d'irrigation comportant, prises séparément ou en combinaison, les caractéristiques énoncée plus haut et telles que definies dans les revendications suivantes.

## Revendications

1. Appareil d'irrigation et d'aspiration pour la chirurgie endoscopique, du type comprenant un réservoir (8) de liquide d'irrigation (10), une pompe (24) pour mettre en circulation le liquide dans une cavité opératoire (2), des moyens pour contrôler la pression dans ladite cavité, des moyens (24,28,30,32) maintien en surpression dudit liquide dans ladite cavité, des moyens (20,32,30) de mesure de la dite surpression dans la cavité opératoire, et des moyens (52 à 58,24,18,28) pour contrôler les flux du liquide, l'injection et l'extraction du liquide pouvant être effectuées par l'intermédiaire de l'instrument opératoire (1) ou exploratoire utilisé, caractérisé en ce qu'il comprend en outre :
- des moyens (44,46,48,50) de chauffage et de maintien en température du liquide d'irrigation (10) dans une plage de réglage comprise entre 40° et 50°C et en ce que réglage de la dite surpression est opeté dans une plage comprise entre 5,3 et 10,6 kPa (40 et 80 mm/Hg), afin d'une part de s'opposer au flux sanguin au travers des liaisons dans les vaisseaux lésés et d'autre part, le flux sanguin étant arrété, d'y coaguler le sang par l'effet hémostatique de la température ;

2. Appareil selon la revendication 1, caractérisé en ce qu'il comprend en outre des moyens (56) de mesure différentielle entre les volumes injectés et les volumes récupérés ;

3. Appareil selon la revendication 2, caractérisé en ce que le liquide d'irrigation passe successivement du réservoir (8), dans une pompe péristaltique (24), puis dans la cavité (2) par l'intermédiaires de l'instrument (1), puis dans une cellule piézométrique (20) mesurant la surpréssion dans la cavité, puis dans une vanne (18) de contrôle de la pression intracavitaire, pour être finalement receptionné dans une enceinte de reception (16) en dépression ;

4. Appareil selon la revendication 3, caractérisé en ce que l'entrainement (26) de ladite pompe péristaltique est placé sous la dépendance à la fois de moyens manuels (28,30) de réglage du débit, et de moyens automatiques (34) d'arrêt en cas de surpression dans la cavité, et en ce que la dite vanne (18) de contrôle de la pression intracavitaire est placée sous la dependance des moyens piézométriques (20,32) de mesure de la surpression dans la cavité ;

5. Appareil selon la revendication 3, caractérisé en ce que ladite vanne de réglage est du type à écrasement de tuyau par un doigt presseur (34), le dit doigt presseur étant mû par un vérin pneumatique (36) actionné en ouverture par la dépression régnant dans ladite enceinte, et en fermeture par un ressort (40) agissant à l'encontre de la dépression ;

6. Appareil selon la revendication 3, caractérisé en ce que les dits moyens de chauffage et/ou de maintien en température comprennent un bain-marie (42) dans lequel baigne ledit réservoir (8) de contention du liquide d'irrigation ;

7. Appareil selon la revendication 6, caractérisé en ce qu'un moyen de chauffage dudit bain-marie est constitué par une résistance électrique (44) placée sous la dépendance d'un thermostat de régulation (46,50) avec un seuil bas de déclenchement et un seuil haut d'arrêt distants en température d'environ 2°C .

8. Appareil selon la revendication 3, caractérisé en ce que le tube souple continu (6) relie le reservoir (8) à un ajutage d'entrée (4) dudit instrument (1), le dit tube souple continu constituant le tuyau de la pompe péristaltique (24);

9. Appareil selon la revendication 3, caractérisé en ce que ledit réservoir (8) est une poche souple ;

10. Appareil selon les revendications 8 et 9, caractérisé en ce que le tube souple (6) destiné à relier la poche (8) à l'instument, constitue avec la poche un conditionnement stérile du liquide d'irrigation, et en ce qu'un tube souple (14) reliant l'enceinte de réception (16) à la dite vanne de contrôle (18), d'une part, et à une pompe à vide (22) d'autre part, constitué avec l'enceinte un conditionnement stérile du liquide d'irrigation récupéré.

## Claims

1. Irrigation and suction apparatus for endoscopic surgery, of the type comprising a reservoir (8) of irrigation liquid (10), a pump (24) for circulating the liquid in an operating cavity (2), means for controlling the pressure in the said cavity, means (24,28,30,32) for maintaining the said liquid under super pressure in the said cavity (2), means (20) for measuring the said supra pressure in the operating cavity, and means (52 to 58,24,18,28) for controlling the flow of liquid, injection and removal of the liquid to be carried out with the aid of the operating instrument (1) or an exploratory instrument used, characterised in that it additionally comprises means (44,46,48,50) for heating and maintaining the temperature of the irrigation liquid (10) in a range of adjustment comprising between 40 and 50°C, and in that the said over supra pressure is maintained in a range between 5.3 and 10.6 KPa (40 to 80 mm/Hg), in order, on the one hand, to oppose the blood flow through connections in the damaged vessels and, on the other hand, when the blood flow has been stopped, to coagulate the blood by means of the haemostatic effect of temperature.

2. Apparatus according to Claim 1, characterised in that it additionally comprises: means (56) for differential measurement between the injected volume and recovered volume.

3. Apparatus according to Claim 2, characterised in that the irrigation liquid passes successively from the reservoir (8), into a peristaltic pump (24), then into the cavity (2) by means of the instrument (1), then into a piezometric cell (20) measuring the super pressure in the cavity, then into a valve (18) for controlling the pressure inside the cavity, and is finally received in a low pressure receptacle (16).

4. Apparatus according to Claim 3, characterised in that the drive (26) of the said peristaltic pump takes place under the control of both manual means (28,30) for regulating the flow rate and automatic stop means (34) in the case of supra pressure in the cavity, and in that the said valve (18) for controlling the pressure inside the cavity operates under the control of piezometric means (20,32) for measuring the super pressure in the cavity.

5. Apparatus according to Claim 3, characterised in that the said regulating valve is of the type involving squeezing of a pipe by a spring-finger (34), the said spring-finger being moved by a pneumatic jack (36) acted upon in the open position by the low pressure prevailing in the said chamber, and in the closed position by a spring (40) acting counter to the low pressure.

6. Apparatus according to Claim 3, characterised in that the said means for heating and/or maintaining the temperature comprise a waterbath (42) in which floats the said reservoir (8) containing the irrigation liquid.

7. Apparatus according to Claim 6, characterised in that one means for heating the said waterbath consists of an electrical resistor (44) under the control of a regulating thermostat (46,50) with a bottom trigger threshold and a top limit threshold approximately 2°C apart in temperature.

8. Apparatus according to Claim 3, characterised in that a continuous flexible tube (6) connects the reservoir (8) to an inlet nozzle (4) on the said instrument (1), the said continuous flexible tube constituting the pipe of the peristaltic pump (24).

9. Apparatus according to Claim 3, characterised in that the said reservoir (8) is a flexible bag.

10. Apparatus according to Claims 8 and 9, characterised in that the flexible tube (6) to connect the bag (8) to the instrument constitutes, together with the bag, a sterile pack of the irrigation liquid, and in that a flexible tube (14) connecting the receiving chamber (16) to the said control valve (18), on the one hand, and to a vacuum pump (22) on the other hand, constitutes, together with the chamber, a sterile pack of the recovered irrigation liquid.

## Patentansprüche

1. Vorrichtung zum Bespülen und Beatmen für die endoskopische Chirurgie, mit einem Vorratsbehälter (8) für Spülflüssigkeit (10), einer Pumpe (24) zum Umwälzen der Flüssigkeit in einer Operationshöhlung (2), mit Mitteln zum Regeln des Druckes in der Höhlung, mit Mitteln (24,28,30,32) zum Aufrechterhalten eines Überdruckes der Flüssigkeit in der Höhlung, mit Mitteln (20) zum Messen des Überdruckes in der Höhlung, und mit Mitteln (52 bis 58,24,18,28) zum Regeln des Durchsatzes der Flüssigkeit, wobei das Einführen und Abziehen der Flüssigkeit mittels Operationsinstrumenten oder angewandter Hilfsmittel durchgeführt werden können,
dadurch gekennzeichnet, daß die Vorrichtung u.a. umfaßt:
- Mittel (44, 46, 48, 50) zum Erwärmen und zum Halten der Temperatur der Spülflüssigkeit (10) in einer Regeleinrichtung zwischen 40 und 50°C, wobei die Regelung des genannten Überdruckes in einer Einrichtung betrieben wird, die zwischen 5,3 und 10,6 KP (40 und 80 mm/Hg) vorgenommen wird, um einerseits den Blutfluß in den Verbindungen zwischen dem verletzten Gewebe zu behindern und andererseits bei angehaltenem Blutfluß das Blut aufgrund der hämostatischen Wirkung der Temperatur gerinnen zu lassen.

2. Vorrichtung nach Anspruch 1, dadurch gekennzeichnet, daß diese u.a. Mittel (56) für eine differentielle Messung zwischen den eingeführten und den zurückgeführten Volumina aufweist.

3. Vorrichtung nach Anspruch 2, dadurch gekennzeichnet, daß die Spülflüssigkeit sukzessive aus dem Reservoir (8) in eine peristaltische Pumpe (24) strömt, sodann in die Höhlung (2) mittels des Instrumentes (1), sodann in eine piezometrische Zelle (20), die den Überdruck in der Höhlung mißt, sodann in ein Regelventil (18) des Druckes in der Höhlung, um schließlich in einer unter Unterdruck stehenden Aufnahmekammer (16) aufgenommen zu werden.

4. Vorrichtung nach Anspruch 3, dadurch gekennzeichnet, daß der Antrieb (26) der peristaltischen Pumpe von manuellen Mitteln (28, 30) zum Regeln des Durchsatzes sowie von automatischen Mitteln (34) zum Abstellen im Falle eines Überdruckes in der Höhlung abhängt, und daß das Ventil (18) zum Regeln des Druckes in der Höhlung von piezometrischen Mitteln (20, 32) zum Messen des Überdruckes in der Höhlung abhängt.

5. Vorrichtung nach Anspruch 3, dadurch gekennzeichnet, daß das Regelventil ein Ventil zum Zusammendrücken eines Schlauches mit einem Quetschelement (34) ist, und daß das Quetschelement betätigt wird von einer pneumatischen Hubeinrichtung (36), die im Sinne des Öffnens durch den in der Kammer herrschenden Unterdruck betätigt wird, und im Sinne des Schließens durch eine Feder (40), die entgegen dem Unterdruck wirkt.

6. Vorrichtung nach Anspruch 3, dadurch gekennzeichnet, daß die Mittel zum Erwärmen und zum Halten der Temperatur ein Wasserbad (42) sind, in welchem sich das Reservoir (8) der Spülflüssigkeit befindet.

7. Vorrichtung nach Anspruch 6, dadurch gekennzeichnet, daß ein Heizmittel des Wasserbades aus einem elektrischen Widerstand (44) besteht, der von einem Thermostat (46, 50) betätigt wird, mit einem unteren Auslösewert und einem oberen Anhaltewert der Temperatur mit einem Unterschied von etwa 2°C.

8. Vorrichtung nach Anspruch 3, dadurch gekennzeichnet, daß ein weicher, kontinuierlicher Schlauch (6) das Reservoir (8) mit einem Einlaßstutzen (4) des Instrumentes (1) verbindet, und daß der Schlauch aus dem Anschluß der peristaltischen Pumpe (24) besteht.

9. Vorrichtung nach Anspruch 3, dadurch gekennzeichnet, daß das Reservoir (8) eine nachgiebige Tasche ist.

10. Vorrichtung nach den Ansprüchen 8 und 9, dadurch gekennzeichnet, daß der weiche Schlauch (6) zum Verbinden der Tasche (8) mit dem Instrument zusammen mit der Tasche einen sterilen Zustand der Spülflüssigkeit herstellt, und daß ein weicher Schlauch (14) die Aufnahmekammer (16) mit dem Regelventil (18) einerseits und mit einer Saugpumpe (22) andererseits verbindet, wobei durch die Kammer ein steriler Zustand der wiedergewonnenen Spülflüssigkeit geschaffen wird.
